(19) Europäisches Patentamt
European Patent Office
Office européen des brevets

(11) Veröffentlichungsnummer: **0 534 423 A1**

# (12) EUROPÄISCHE PATENTANMELDUNG

(21) Anmeldenummer: **92116342.4**

(22) Anmeldetag: **24.09.92**

(51) Int. Cl.[5]: **C07D 489/08**, C07D 307/92, A61K 31/485

(30) Priorität: **27.09.91 DE 4132159**

(43) Veröffentlichungstag der Anmeldung:
**31.03.93 Patentblatt 93/13**

(84) Benannte Vertragsstaaten:
**AT BE CH DE DK ES FR GB GR IE IT LI LU NL PT SE**

(71) Anmelder: **BOEHRINGER INGELHEIM KG**

**W-6507 Ingelheim am Rhein(DE)**
(84) **BE CH DE DK ES FR GR IE IT LI LU NL PT SE AT**

(71) Anmelder: **BOEHRINGER INGELHEIM INTERNATIONAL G.M.B.H.**

**W-6507 Ingelheim am Rhein(DE)**
(84) **GB**

(72) Erfinder: **Merz, Herbert, Dr. Dipl.-Chem.**
**Rotweinstrasse 53**
**W-6507 Ingelheim am Rhein(DE)**
Erfinder: **Wiedemann, Ingrid, Dr.**
**Dudenstrasse 16**
**W-6200 Wiesbaden(DE)**
Erfinder: **Ensinger, Helmut, Dr. Dipl.-Chem.**
**Magdeburger Strasse 54**
**W-6507 Ingelheim am Rhein(DE)**
Erfinder: **Stockhaus, Klaus, Dr.**
**Pfarrer-Heberer-Strasse 35**
**W-6530 Bingen(DE)**
Erfinder: **Grauert, Matthias, Dr. Dipl.-Chem.**
**In der Dörrwiese 62**
**W-6507 Ingelheim am Rhein(DE)**

(54) **14-Hydroxy-N-(2-methoxyethyl)-7,8-dihydromorphin und - norisomorphin, Verfahren zu deren Herstellung und deren Verwendung als Arzneimittel.**

(57) Die vorliegende Erfindung betrifft 14-Hydroxy-N-(2-methoxyethyl)-7,8-dihydromorphin und -norisomorphin, Verfahren zu deren Herstellung und deren Verwendung als Arzneimittel.

EP 0 534 423 A1

14-Hydroxy-N-(2-methoxyethyl)-7,8-dihydromorphin und -norisomorphin, Verfahren zu deren Herstellung und deren Verwendung als Arzneimittel.

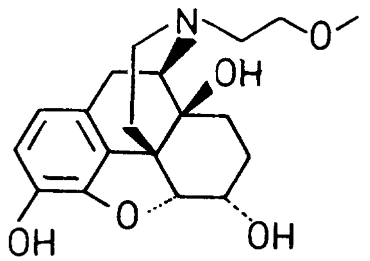

Ia

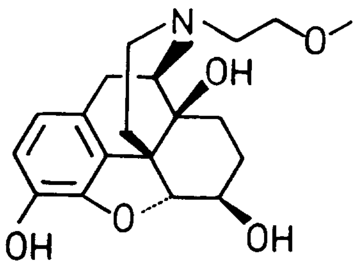

Ib

I

Die vorliegende Erfindung betrifft die epimeren Verbindungen der allgemeinen Formel Ia und Ib - namentlich 14-Hydroxy-N-(2-methoxyethyl)-7,8-dihydromorphin (Ia) und 14-Hydroxy-N-(2-methoxyethyl)-7,8-dihydronorisomorphin (Ib), Verfahren zu deren Herstellung und deren Verwendung als Arzneimittel.

Gegenstand der Erfindung sind ferner die entsprechenden physiologisch geeigneten Säureadditionssalze mit anorganischen oder organischen Säuren. Bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Milchsäure, Malonsäure, Bernsteinsäure, Maleinsäure, Fumarsäure, Äpfelsäure, Weinsäure, Zitronensäure oder Benzoesäure.

Die erfindungsgemäßen Verbindungen sowie deren Säureadditionssalze besitzen wertvolle pharmakologische Eigenschaften und üben eine therapeutisch nutzbare Wirkung auf das Zentralnervensystem aus und können als suchtfreie Analgetika in der Schmerzbekämpfung eingesetzt werden.

Die analgetische Wirkung der erfindungsgemäßen Verbindungen läßt sich im Phenylchinon Writhing-Test belegen, der gemäß S. Irwin [Psychopharmacologia 13, 222 bis 257 (1968)] durchgeführt wurde. In diesem Test besitzt die erfindungsgemäße Verbindung 1a eine mittlere effektive Dosis (MED) von 2,5 mg/kg p.o. (64 % Effekt) und die erfindungsgemäße Verbindung 1b eine MED von 10 mg/kg p.o. (71 % Effekt). Ibuprofen als Referenzverbindung besitzt bei einer Dosis von 25 mg/kg p.o. einen Effekt von 65 %. Im Opiatrezeptorbindungstest mit $[^3H]$-Naloxon als Radioligand besitzt 1a eine $K_i$ von 2,14 nM und unter Zusatz von 0,1 M Natriumchlorid eine $K_i$ von 2,39 nM, was einem Natrium-shift von 1,12 entspricht. Die Verbindung 1b besitzt in diesen beiden Bindungstests einen $K_i$-Wert von 1,07 nM bzw. 1,19 nM (Natrium-shift: 1,11). Dieser geringe Natrium-shift weist auf den antagonistischen Charakter der erfindungsgemäßen Verbindungen hin. Im Opiatrezeptorbindungstest mit $[^3H]$-Dihydromorphin als Radioligand besitzt 1a eine $K_i$ von 0,91 nM und 1b einen $K_i$ von 1,79 nM.

Die erfindungsgemäßen Verbindungen können nach verschiedenen - an sich bekannten - Verfahren durch Reduktion von N-(2-Methoxyethyl)-noroxymorphon hergestellt werden.

Die Herstellung dieses Noroxymorphonderivates ist aus der Deutschen Offenlegungsschrift 32 20 831 bekannt.

Die Reduktion des N-(2-Methoxyethyl)-noroxmorphonhydrochlorids erfolgt nach an sich - aus dem Stand der Technik - bekannten Methoden [R.C. Larock, Comprehensive Organic Transformations, A Guide to Functional Group Preparations, VCH Verlagsgesellschaft m.b.H., Weinheim, 1989, S. 527 ff und zit. Lit.; J. March, Advanced Organic Chemistry, J. Wiley & Sons, New York, 1985, S. 809 und zit. Lit.; C. Ferri, Reaktionen der organischen Synthese, Georg Thieme Verlag, Stuttgart 1978, S. 102, 108, 115 und zit. Lit.].

Bevorzugt wird die Reduktion mit komplexen Alkalibor- oder Alkalialuminiumhydriden - gegebenenfalls in Gegenwart eines Katalysators [A. Hajos, Complex Hydrides, Elsevier, New York 1979; H.O. House, Modern Synthetic Reactions, $2^{nd}$ ed., W.A. Benjamin, New York, 1972, S. 49 ff.; O.H. Wheeler in Patai, The Chemistry of the Carbonyl Group, part 1, Interscience, New York, 1966, S. 507 ff., H.C. Brown, J. Chem. Educ. 38 (1961) 173; E. Schenker, Angew. Chem. 73 (1961) 81; E. Schenker, Newer Methods Prep. Org. Chem. 4 (1968) 196].

Besonders bevorzugt werden Natriumtetrahydridoboranant (Natriumborhydrid) sowie Lithiumtetrahydridoalanat als Reduktionsmittel eingesetzt.

Als Lösungsmittel eignen sich für die Durchführung der Reduktion alle inerten Lösungsmittel, die sich unter den gegebenen Reaktionsbedingungen nicht verändern. Hierzu gehören beispielsweise Ether - wie z.B. Di-n-butylether, Glykoldimethylether (Glyme), Diglykoldimethylether (Diglyme) cyclische Ether - wie z.B. Dioxan und Tetrahydrofuran, sowie - soweit es das eingesetzte Reduktionsmittel zuläßt - bevorzugt Alkohole - wie Methanol, Ethanol, Isopropanol oder Glykol - wobei eine Mischung aus Ethanol und zu Natronlauge besonders bevorzugt wird. - Es ist auch möglich, Gemische der genannten Lösungsmittel zu verwenden.

Die Reduktion selbst erfolgt in einem Temperaturbereich von -10 ° C bis zum Siedepunkt der Reaktionsmischung - bevorzugt zwischen -5 und 40 ° C und besonders bevorzugt zwischen 0 und 30 ° C.

Zur Durchführung der Reduktion wird in einer bevorzugten Ausführungsform das N-(2-Methoxyethyl)-noroxymorphonhydrochlorid in einer Ethanol/Natronlauge-Mischung gelöst und mit dem Reduktionsmittel - ganz besonders bevorzugt Natriumborhydrid - versetzt. Nach Beendigung der Reduktionsreaktion und Isolierung der Reaktionsprodukte werden diese vermittels Säulenchromatographie gereinigt, in die beiden Epimeren getrennt und isoliert.

Eine weitere bevorzugte Ausführungsform besteht in der katalytischen Reduktion mit Wasserstoff. Die Durchführung derartiger katalytischer Reduktionen ist aus dem Stand der Technik bekannt [P.N. Rylander, "Catalytic Hydrogenation over Plantinum Metals", Academic Press, New York, 1967, S. 238]. Bevorzugt wird Raney Nickel als Katalysator eingesetzt [J.A. Schreifels, P.C. Maybury und W.E. Swartz Jr., J. Org. Chem. 46 (1981) 1263].

Bei den genannten Reduktionsreaktionen fallen Gemische der beiden Epimeren an. - Dagegen kann beim Einsatz von Formamidinsulfinsäure [N. Chatterjie und H. Blumberg, J. Med. Chem. 18 (1975) 490] als Reduktionsmittel gezielt das β-Epimere dargestellt werden.

Die nachfolgenden Beispiele sollen die Erfindung erläutern, ohne sie auf den Bereich der Beispiele einzuschränken:

## Beispiele

1) 7,64 g (0,02 Mol) N-(2-Methoxyethyl)-noroxymorphon-hydrochlorid werden in 150 ml Ethanol suspendiert und unter Rühren mit 10,5 ml 2N Natronlauge versetzt, wobei man eine Lösung erhält. Unter weiterem Rühren wird mit 0,41 g 98 %-igem (0,01 Mol) Natriumborhydrid versetzt. Man läßt 1 h bei Raumtemperatur nachrühren und versetzt mit 50 ml Wasser. Anschließend zieht man das Lösungsmittel i.Vak. bis auf einen Rückstand von ca. 60 g ab und läßt 2 h im Eisbad kristallisieren. Die ausgefallenen Kristalle werden abgesaugt, dreimal mit jeweils 10 ml Wasser gewaschen und anschließend getrocknet. Die Mutterlauge wird auf ca. 30 g weiter eingeengt und erneut unter Eiskühlung kristallisiert. Es wird abgesaugt; die Kristalle werden mit Wasser gewaschen und getrocknet.

Das Kristallisat wird durch Säulenchromatographie an 1 kg Kieselgel (MN K 60, 230-400 mesh ASTM, von Macherey und Nagel) unter Verwendung einer Mischung aus Chloroform, Methanol und konz. Ammoniak (90 : 10 : 0,5) als Fließmittel in die beiden Epimeren getrennt. Man engt die geeigneten Fraktionen i.Vak. ein und kristallisiert den Rückstand aus Ethanol um. Man erhält 1,88 g (27 %) 14-Hydroxy-N-(2-methoxyethyl)-7,8-dihydronormorphin (α-Epimer) als schneller laufendes Isomer vom Schmp. 192-194 ° C und 1,13 g (16 %) 14-Hydroxy-N-(2-methoxyethyl)-7,8-dihydronorisomorphin (β-Epimer) vom Schmp. 201-203 ° C.

Die beiden Epimere weisen folgende Drehwerte auf:

$[\alpha]_D^{25}$ (α-Epimer): -155,3 ° ,

(c = 1, $CH_3OH$/1N HCl, 1:1) sowie $[\alpha]_D^{25}$ (β-Epimer): -130,2 ° (c = 1, $CH_3OH$/1N HCl 1:1).

2) 7,64 g (0,02 Mol) N-(2-Methoxyethyl)-noroxymorphonhydrochlorid werden unter Stickstoffatomosphäre in 400 ml Wasser gelöst und unter Rühren mit 11 ml 2 N Natronlauge versetzt. Man erhält eine klare Lösung, zu der man eine Lösung von 8,64 g (0,08 Mol) Formamidinsulfinsäure in 70 ml 2N Natronlauge zufügt. Daraufhin läßt man 1 h bei 85 ° C rühren, anschließend auf Raumtemperatur abkühlen und versetzt mit 5 Tropfen konz. Salzsäure und einer Lösung von 10 g Natriumcarbonat und 10 g Natriumhydrogencarbonat in 100 ml Wasser. Die wässrige Phase wird sechsmal mit je 150 ml Dichlormethan extrahiert. Die vereinigten organischen Extrakte werden getrocknet und das Lösungsmittel wird nach dem Abfiltrieren des Trockenmittels i.Vak. abgezogen. Der Rückstand wird aus ca. 100 ml Ethanol umkristallisiert. Man erhält so 5,0 g (72 %) des 14-Hydroxy-N-(2-methoxyethyl)-7,8-dihydronorisomorphins (β-Epimer), Schmp.: 201 ° C.

Zur vorliegenden Erfindung gehören auch pharmazeutische Zubereitungen, die neben nicht-toxischen, inerter pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur

Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben die üblichen Trägerstoffen enthalten, wie (a) Füll- und Streckmittel, z.B. Stärken, Milchzucker, Rohrzucker, Glukose Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resportionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebener Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{15}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummethahydroxid, Bentonit. Agar-Agar und Tragant oder Gemische dieser Stoffe enthalten.

Die genannten Formulierungsformen können auch Färbemittel, Konservierungsstoffe sowie geruchs und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können oral, rektal, parenteral (intravenös, intramuskulär, subkutan) angewendet werden. Als geeignete Zubereitungen kommen Injektionslösungen, Lösungen und Suspensionen für die Therapie in Frage.

Im allgemeinen hat es sich in der Humanmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse

zu verabreichen. Eine Einzelgabe enthält den oder die erfindungsgemäßen Wirkstoffe von vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die nachfolgenden Herstellungsbeispiele sollen die Erfindung erläutern, ohne sie einzuschränken:

## Tabletten

1. Die Tablette enthält folgende Bestandteile:

| | |
|---|---|
| Wirkstoff gemäß Formel 1 | 0,020 Teile |
| Stearinsäure | 0,0I0 " |
| Dextrose | I,890 " |
| gesamt | 1,920 Teile |

Herstellung:
Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen:

| | |
|---|---|
| Wirkstoff gemäß Formel 1 | 50 mg |
| Neribas Salbe (Handelsware Scherax) | ad 10 g |

Herstellung:
Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme

| Zusammensetzung: | |
|---|---|
| Wirkstoff gemäß Formel 1 | 50 mg |
| Neribas Salbe (Handelsware Scherax) | ad 10 g |

Herstellung
Der Wirkstoff wird mit 0,5 g Cremegrundlage verrieben und die restliche Grundlage in Teilmengen zu 1,0 g nach und nach mit dem Pistill eingearbeitet. Man erhält eine 0,5%ige Creme. Die Verteilung des Wirkstoffes in der Grundlage wird optisch unter dem Mikroskop kontrolliert.

4. Ampullenlösung

| Zusammensetzung: | |
|---|---|
| Wirkstoff gemäß Formel 1 | 1,0 mg |
| Natriumchlorid | 45,0 mg |
| Aqua pro inj. | ad 5,0 ml |

Herstellung:
Der Wirkstoff wird bei Eigen-pH oder gegebenenfalls bei pH 5,5 bis 6,5 in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 10 mg Wirkstoff.
5. Suppositorien Jedes Zäpfchen enthält:

| | |
|---|---|
| Wirkstoff gemäß Formel 1 | 1,0 Teile |
| Kakaobutter (Schmp.: 36-37 ° C) | 1200,0 Teile |
| Carnaubawachs | 5,0 Teile |

Herstellung
Kakaobutter und Carnaubawachs werden zusammengeschmolzen. Bei 45 ° C gibt man den Wirkstoff hinzu und rührt, bis eine komplette Dispersion entstanden ist.
Die Mischung wird in Formen entsprechender Größe gegossen und die Zäpfchen zweckmäßig verpackt.

## Patentansprüche

1. Morphinderivate der allgemeinen Formel I

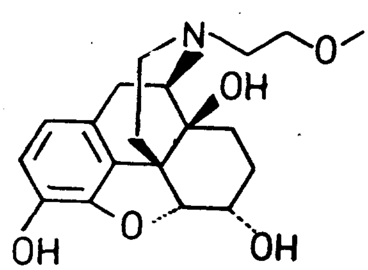

Ia

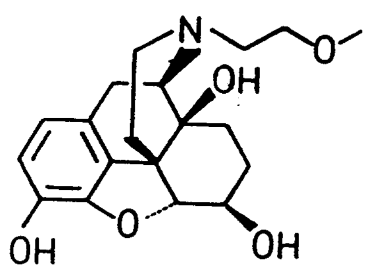

Ib

I

2. Pharmazeutische Zubereitung, gekennzeichnet durch einen Gehalt an einer Verbindung nach Anspruch 1.

3. Verwendung von Verbindungen nach Anspruch 1 und deren Säureadditionssalze bei der Herstellung von pharmazeutischen Zubereitungen mit schmerzbekämpfender Wirkung.

4. Verfahren zum Herstellen von Verbindungen der allgemeinen Formel I, dadurch gekennzeichnet, daß man N-(2-Methoxyethyl)noroxymorphon mit einem Reduktionsmittel umsetzt, das Reaktionsgemisch aufarbeitet und das/die Reaktionsprodukt(e) isoliert.

5. Verfahren nach Anspruch 4, dadurch gekennzeichnet, daß das Reduktionsmittel ein komplex Hydrid eines Elementes der dritten Hauptgruppe darstellt.

6. Verfahren nach Anspruch 5, dadurch gekennzeichnet, daß man als Reduktionsmittel Natriumtetrahydridoboranat (Natriumborhydrid) verwendet.

7. Verfahren nach Anspruch 5, dadurch gekennzeichnet, daß man als Reduktionsmittel Lithiumtetrahyridoalanat (Lithiumaluminiumhydrid verwendet).

8. Verfahren nach Anspruch 4, dadurch gekennzeichnet, daß man die Reduktion mit Wasserstoff in Gegenwart eines Katalysators durchführt.

9. Verfahren nach Anspruch 8, dadurch gekennzeichnet, daß der Katalysator Raney Nickel ist.

10. Verfahren nach Anspruch 4, dadurch gekennzeichnet, daß man als Reduktionsmittel Formamidinsulfinsäure einsetzt.

Europäisches Patentamt

# EUROPÄISCHER RECHERCHENBERICHT

Nummer der Anmeldung

EP 92 11 6342
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A<br>D | EP-A-0 096 279 (BOEHRINGER INGELHEIM K.G.)<br>* Zusammenfassung *<br>& DE-A-3 220 831<br>--- | 1-10 | C07D489/08<br>C07D307/92<br>A61K31/485 |
| A | CHEMICAL ABSTRACTS, vol. 62, no. 5, 1. März 1965, Columbus, Ohio, US; abstract no. 5308h, U. WEISS ET AL.<br>* Zusammenfassung *<br>& JOURNAL OF MEDICINAL CHEMISTRY Bd. 8, Nr. 1, 1965, WASHINGTON US Seiten 123 - 125<br>--- | 6 | |
| A | FR-M-6 112 (ENDO LABORATORIES INC.)<br>* Seite 2 *<br>--- | 7 | |
| A | JOURNAL OF ORGANIC CHEMISTRY Bd. 40, Nr. 1, 1975, EASTON US Seiten 31 - 34 E.F. HAHN ET AL.<br>* Zusammenfassung *<br>--- | 5,6 | RECHERCHIERTE SACHGEBIETE (Int. Cl.5) |
| A | RES. COMM. CHEM. PATHOL. PHARMACOL. Bd. 12, Nr. 1, 1975, COLUMBUS, OHIO Seiten 43 - 65 L. MALSPEIS ET AL.<br>* Seite 46 *<br>--- | 6 | C07D<br>A61K |
| A,D | JOURNAL OF MEDICINAL CHEMISTRY Bd. 18, Nr. 5, 1975, WASHINGTON US Seiten 490 - 492 N. CHATTERJIE ET AL.<br>* Zusammenfassung *<br>--- | 10 | |
| A | LIFE SCIENCES Bd. 17, Nr. 3, 1975, Seiten 465 - 475 S.H. POLLOCK<br>* Zusammenfassung *<br>--- | 4 | |

Der vorliegende Recherchenbericht wurde für alle Patentansprüche erstellt

| Recherchenort | Abschlußdatum der Recherche | Prüfer |
|---|---|---|
| BERLIN | 16 DEZEMBER 1992 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 11 6342
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | J. PHARMACOL. EXP. THER.<br>Bd. 200, Nr. 3, 1977,<br>Seiten 496 - 500<br>A.Z. RONAI ET AL.<br>* Zusammenfassung *<br>----- | 1-10 | |

RECHERCHIERTE SACHGEBIETE (Int. Cl.5 )

Der vorliegende Recherchenbericht wurde für alle Patentansprüche erstellt

| Recherchenort | Abschlußdatum der Recherche | Prüfer |
|---|---|---|
| BERLIN | 16 DEZEMBER 1992 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)